# EUROPÄISCHE PATENTSCHRIFT

(11) **EP 0 555 698 B1**
(45) Veröffentlichungstag und Bekanntmachung des Hinweises auf die Patenterteilung: **08.05.1996**
(21) Anmeldenummer: 93101289.2
(22) Anmeldetag: 28.01.1993
(51) Int. Cl.: C07C 45/43, C07C 47/55, C07C 205/44, C07C 47/54, C07C 303/22, C07C 309/44

(54) **Verbessertes Verfahren zur Herstellung von gegebenenfalls substituierten Benzaldehyden**
Process for the preparation of possibly substituted benzaldehydes
Procédé de préparation de benzaldéhydes éventuellement substitués

(30) Priorität: 10.02.1992 DE 4203792
(43) Veröffentlichungstag der Anmeldung: 18.08.1993
(73) Patentinhaber: BAYER AG, D-51368 Leverkusen (DE)
(72) Erfinder: Broda, Witold Dr., W-5206 Neunkirchen (DE); Beitzke, Bernhard, Dr., W-5064 Rösrath (DE); Richter, Hartmut, Dr., W-4018 Langenfeld (DE); Fiege, Helmut, Dr., W-5090 Leverkusen 1 (DE)

(56) Entgegenhaltungen:
- DE-A- 2 752 612
- DE-A- 3 142 856
- FR-A- 2 265 722

## Beschreibung

Die vorliegende Erfindung betrifft ein verbessertes Verfahren zur Herstellung von gegebenenfalls substituierten Benzaldehyden durch Hydrolyse der entsprechenden Benzalchloride.

Benzaldehyde sind wichtige Zwischenprodukte zur Herstellung von Farbstoffen, Pharmazeutika und Pflanzenschutzmitteln. Es ist bekannt, daß man Benzaldehyde durch Verseifung der entsprechenden Benzalchloride herstellen kann (siehe z.B. Houben-Weyl, Methoden der organischen Chemie, Band E3, S. 350 ff. (1983)). Der Einsatz von Schwefelsäure und Metallsalzen hat sich dabei als vorteilhaft erwiesen. Es ist bisher jedoch nicht bekannt geworden, daß man die Schwefelsäure wiederverwenden kann. Die Wiederverwendung der Schwefelsäure hat auch nicht nahegelegen, da sie bei der Hydrolyse von Benzalchloriden organische Stoffe aufnimmt und deshalb bei der Rückführung eine Akkumulation dieser Stoffe zu befürchten oder eine spezielle Aufbereitungsanlage zur Reinigung der rückzuführenden Schwefelsäure vorzusehen war. Außerdem sind die bekannten Verfahren zur Herstellung von Benzaldehyden hinsichtlich der Reaktionsführung sowie wegen des Einsatzes von Metallsalzen und des Anfalls von verdünnter Schwefelsäure nach nicht optimal.

Aus DE-A 2 752 612, DE-A 3 142 856 und FR-A 2 265 722 sind Verseifungen von Benzalchloriden zu Benzaldehyden bekannt, bei denen mit Salzsäure oder Schwefelsäure gearbeitet wird. Dort sind jedoch die Wasser- oder Säuremengen um ein vielfaches größer als bei der vorliegenden Erfindung.

Es wurde nun ein Verfahren zur Herstellung von gegebenenfalls substituierten Benzaldehyden durch Verseifung der entsprechenden Benzalchloride mit Wasser in Gegenwart von Schwefelsäure gefunden, das dadurch gekennzeichnet ist, daß man in Gegenwart von 100 bis 250 Mol-% Wasser, bezogen auf verseifbare Chloratome arbeitet und 1 bis 40 Gew.-% Schwefelsäure (gerechnet als 100%ige Schwefelsäure), bezogen auf Benzalchlorid einsetzt, Wasser in Gegenwart von Schwefelsäure bei 20 bis 160°C vorlegt, das Benzalchlorid und gegebenenfalls einen Teil des Wassers bei dieser Temperatur zumischt, nach Beendigung der Reaktion die Säurephase von der organischen Phase abtrennt, die Säurephase ganz oder teilweise in den nächsten Ansatz zurückführt und aus der organischen Phase den erhaltenen Benzaldehyd gewinnt.

Mit dem erfindungsgemäßen Verfahren kann man beispielsweise aus Benzalchloriden der Formel (I) 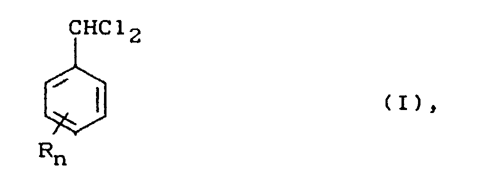
in der
- R: für Fluor, Chlor, Brom, NO₂ und/oder SO₃H und
- n: für Null, 1, 2, 3 oder 4 steht,
Benzaldehyde der Formel (II) 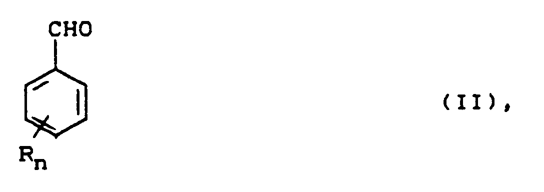
in der
R und n die bei Formel (I) angegebene Bedeutung haben, herstellen.

Bevorzugt setzt man in das erfindungsgemäße Verfahren unsubstituiertes Benzalchlorid, 2-, 3- oder 4-Chlorbenzalchlorid, 2,3-, 2,4-, 2,5-, 2,6- oder 3,4-Dichlorbenzalchlorid, 4-Fluorbenzalchlorid, 2-Fluor-3-chlorbenzalchlorid oder Tetrachlorbenzalchlorid ein und erhält daraus unsubstituierten Benzaldehyd, 2-, 3- oder 4-Chlorbenzaldehyd, 2,3-, 2,4-, 2,5-, 2,6- oder 3,4-Dichlorbenzaldehyd, 4-Fluorbenzaldehyd, 2-Fluor-3-chlorbenzaldehyd oder Tetrachlorbenzaldehyd.

Besonders bevorzugt setzt man in das erfindungsgemäße Verfahren 2- oder 4-Chlorbenzalchlorid ein und erhält daraus 2- oder 4-Chlorbenzaldehyd.

In das erfindungsgemäße Verfahren kann man die Benzalchloride z.B. als 60 bis 100 % reine Edukte einsetzen. Als Verunreinigungen können sie beispielsweise bis zu 40 Gew.-% des entsprechenden Benzotrichlorids und bis zu 2 Gew.-% des entsprechenden Benzylchlorids enthalten. Vorzugsweise haben die eingesetzten Benzalchloride eine Reinheit von 70 bis 98 Gew.-%, enthalten 2 bis 30 Gew.-% des entsprechenden Benzotrichlorids und weniger als 1 Gew.-% des entsprechenden Benzylchlorids. Besonders bevorzugt sind Benzalchloride, die eine Reinheit von 85 bis 95 Gew.-% aufweisen, 5 bis 15 Gew.-% des entsprechenden Benzotrichlorids und weniger als 0,5 Gew.-% des entsprechenden Benzylchlorids enthalten.

Die vorzulegende Schwefelsäure kann beispielsweise eine Konzentration von 10 bis 90 Gew.-% aufweisen. Vorzugsweise beträgt deren Konzentration 20 bis 80 Gew.-%, besonders bevorzugt 35 bis 70 Gew.-%. Schwefelsäure wird in Mengen von 1 bis 40 Gew.-% (gerechnet als 100 %ige Schwefelsäure), bezogen auf das Benzalchlorid, eingesetzt. Die bevorzugt einzusetzende Schwefelsäuremenge (gerechnet als 100 %ige Schwefelsäure) richtet sich danach, wieviel Wasser die Schwefelsäure enthält und wieviel Wasser dem Reaktionsgemisch während der Reaktion zugeführt wird. Wenn man beispielsweise das gesamte einzusetzende Wasser zusammen mit der Schwefelsäure vorlegt, ist es bevorzugt, 10 bis 25 Gew.-% Schwefelsäure (gerechnet als 100 %ige Schwefelsäure), bezogen auf Benzalchlorid, einzusetzen. Wenn man beispielsweise einen wesentlichen Teil, z.B. über 70 Gew.-%, des gesamten einzusetzenden Wassers während der Reaktion zudosiert, ist es bevorzugt, 5 bis 10 Gew.-% Schwefelsäure (gerechnet als 100 %ige Schwefelsäure), bezogen auf Benzalchlorid, einzusetzen.

Im Reaktionsgemisch muß mindestens soviel Wasser zugegen sein oder zudosiert werden, wie stöchiometrisch zur Verseifung der verseifbaren Chloratome gebraucht wird.

Die Wassermenge beträgt 100 bis 250 Mol-%, bezogen auf verseifbare Chloratome. Bevorzugt ist diese Menge 110 bis 200 Mol-%, besonders bevorzugt 120 bis 180 Mol-%. Wenn die eingesetzte Schwefelsäure nicht soviel Wasser enthält oder während der Reaktion zuviel Wasser entweicht, muß zusätzlich Wasser vorgelegt oder nachdosiert werden. Man kann das gesamte einzusetzende Wasser mit der Schwefelsäure vorlegen, was die bevorzugte Art der Wasserzugabe ist. Dann steigt während der Reaktion die Konzentration der Schwefelsäure an, Man kann auch während der Reaktion Wasser zudosieren und so die Schwefelsäure-Konzentration konstant oder in gewissen Grenzen halten, Man kann auch einen Teil des einzusetzenden Wassers in Form von 10 bis 90 Gew.-%iger Schwefelsäure, gegebenenfalls zusammen mit einem Teil des organischen Materials vorlegen und das restliche Wasser und das restliche organische Material während der Reaktion zudosieren.

Die Temperatur der vorgelegten Schwefelsäure liegt vorzugsweise im Bereich 40 bis 140°C, besonders bevorzugt im Bereich 70 bis 130°C. Zumindest bei einem Teil der Schwefelsäure handelt es sich um aus einem vorangegangenen Ansatz rückgeführte Schwefelsäure. Vorzugsweise wird die gesamte Schwefelsäure im Kreis geführt und nur verbrauchtes Wasser zugefügt.

Die Dosierung des jeweiligen Benzalchlorids erfolgt vorzugsweise in dem Maße, wie es abreagiert. Man kann die Dosierung des jeweiligen Benzalchlorides z.B, über die Rückflußtemperatur des Reaktionsgemisches steuern, beispielsweise so, daß das Benzalchlorid in einem Maße eindosiert wird, daß die Temperatur des Reaktionsgemisches während der Reaktion um nicht mehr als 35°C, vorzugsweise um nicht mehr als 25°C gegenüber der Temperatur vor der Zugabe des Benzalchlorids ansteigt und 180°C nicht übersteigt. Man kann auch die Temperatur bei der Dosierung des Benzalchlorides konstant halten und es gemäß der vorher ermittelten Reaktionsgeschwindigkeit zudosieren. Beispielsweise ist für die Zudosierung des jeweiligen Benzalchlorids ein Zeitraum von 1 bis 20 Stunden vorzusehen.

Es ist vorteilhaft, nach Beendigung der Zugabe des jeweiligen Benzalchlorids das Reaktionsgemisch noch eine Zeit lang, z.B. 1 bis 5 Stunden, bei Reaktionstemperatur zu halten. Man kann dann praktisch den vollständigen Umsatz des eingesetzten Benzalchlorids erreichen.

Die nun vorzunehmende Phasentrennung kann beispielsweise bei 20 bis 160°C, vorzugsweise bei 60 bis 140°C und besonders bevorzugt bei 100 bis 130°C durchgeführt werden. Bei hohen Schwefelsäurekonzentrationen kann die Phasentrennung erschwert sein. Man kann dann vor der Phasentrennung Wasser oder verdünnte Schwefelsäure zufügen, z.B. das für den nächsten Ansatz nachzudosierende Wasser, dadurch die Konzentration der Schwefelsäure erniedrigen, z.B. auf unter 70 Gew.-% und dadurch die Phasentrennung erleichtern.

Die abgetrennte Säurephase wird teilweise oder, bevorzugt, vollständig zum nächsten Ansatz zurückgeführt. Obwohl die abgetrennte Säurephase organische Stoffe enthält, z.B. Benzaldehyde und Benzoesäuren, kann sie überraschenderweise praktisch beliebig oft zurückgeführt werden, ohne daß es darin zu einer Akkumulierung der organischen Stoffe kommt.

Die Gewinnung des hergestellten Benzaldehyds aus der organischen Phase kann auf verschiedene Weise erfolgen. Beispielsweise kann man die organische Phase, gegebenenfalls nach einer Wäsche mit wäßrigem Alkali, destillieren. Man kann die organische Phase gegebenenfalls auch direkt destillieren.

Benzaldehyde mit einem Schmelzpunkt von über 40°C kann man auch isolieren, indem man die abgetrennte organische Phase in Wasser oder eine wäßrige alkalische Lösung mit einer Temperatur von 0 bis 30°C einträgt und den ausfallenden Benzaldehyd durch Filtration abtrennt.

Das erfindungsgemäße Verfahren kann man kontinuierlich und diskontinuierlich durchführen.

Das erfindungsgemäße Verfahren hat eine Reihe von Vorteilen: Es vermeidet den Einsatz von Metallsalzen, es gestattet die benötigte Schwefelsäure vollständig im Kreis zu führen, es liefert Benzaldehyde in nahezu quantitativen Ausbeuten und bei ihm werden eventuell im Einsatzprodukt vorhandene Benzylchloride nicht hydrolysiert. Außerdem gestattet das erfindungsgemäße Verfahren eine umsatzkontrollierte Reaktionsführung, was für das Erreichen einer hohen Verfahrenssicherheit mit geringem Aufwand von Vorteil ist. Das erfindungsgemäße Verfahren ist deshalb besonders wirtschaftlich und umweltfreundlich.

### Beispiele

### Beispiel 1

6,26 Gew.-Teile 40 gew.-%iger Schwefelsäure wurden in einem Reaktionsgefäß vorgelegt, auf 110°C erhitzt und unter Rühren im Verlaufe von 7 Stunden 19,93 Gew.-Teile 4-Chlorbenzalchlorid (95 Gew.-% rein, enthaltend 4,8 Gew.-% 4-Chlorbenzotrichlorid und 0,2 Gew.-% 4-Chlorbenzylchlorid) so zudosiert, daß die Temperatur des Reaktionsgemisches 125°C nicht überstieg. Das entweichende wasserdampfhaltige Chlorwasserstoff-Gas wurde in einem Absorber aufgefangen. Anschließend wurde noch 5 Stunden bei 125 bis 130°C nachgerührt. Danach wurde bei 125°C die organische Phase von der Säurephase abgetrennt. Die Säurephase (ca. 60 gew.-%ige Schwefelsäure) wurde vollständig zum nächsten Ansatz zurückgeführt. Die organische Phase wurde mit wäßriger Natronlauge gewaschen und anschließend destilliert. Es wurden 13,34 Gew.-Teile 4-Chlorbenzaldehyd (Reinheit 100 %) erhalten.

### Beispiel 2

In einem Reaktor wurden 1.200 g 60 gew.-%ige Schwefelsäure vorgelegt und auf 120°C erhitzt. Bei 120°C wurden unter ständigem Rühren innerhalb von 6 Stunden 3.100 g eines Gemisches aus 94,4 Gew`-% 2-Chlorbenzalchlorid, 5,1 Gew.-% 2-Chlorbenzotrichlorid und 0,2 Gew.-% 2-Chlorbenzylchlorid zugepumpt. Danach wurde das Reaktionsgemisch 2 Stunden bei 110°C nachgerührt, was einen vollständigen Umsatz des 2-Chlorbenzalchlorids ergab. Nach der Zugabe von 270 g Wasser wurden die resultierenden Phasen bei 110°C getrennt. Man erhielt 1.167 g 62,7 gew.-%ige Schwefelsäure, die in die darauffolgende Partie zurückgeführt wurde und 2.225 g 2-Chlorbenzaldehyd (91 gew.-%ig).

Das Beispiel wurde dreimal wiederholt, d.h. die einmal eingesetzte Schwefelsäure dreimal zurückgeführt. Die durchschnittliche Rückgewinnungsrate der Schwefelsäure betrugt dabei 99 Gew,-% vom Einsatz und die durchschnittliche Aldehydausbeute 98 % der Theorie.

### Beispiel 3

In einem Reaktor wurden 1.015 g 60 gew.-%ige Schwefelsäure vorgelegt und auf 136°C erhitzt. Innerhalb von 7 Stunden wurden unter Rühren 3 250 g eines Gemisches aus 97,1 Gew.-% 2,4-Dichlorbenzalchlorid, 0,77 Gew.-% 2,4-Dichlorbenzotrichlorid und 0,35 Gew.-% 2,4-Dichlorbenzylchlorid mit konstanter Geschwindigkeit zugepumpt. Dabei wurde die Temperatur bis auf 140°C gesteigert. Nach einer weiteren Stunde bei 140°C war der Umsatz quantitativ. Nach der Zugabe von 255 g Wasser wurden die resultierenden Phasen bei 140°C getrennt. Es wurden 982 g 57,7 gew.-%ige Schwefelsäure und 2.512 g Rohaldehyd erhalten. Die Destillation des rohen Aldehyds ergab 2.368 g 98 gew.-%igen 2,4-Dichlorbenzaldehyd.

Das Beispiel wurde wiederholt, wobei die abgetrennte Schwefelsäure zurückgeführt wurde. Die Rückgewinnungsrate der Schwefelsäure betrugt 96 Gew.-% des Einsatzes.

### Beispiel 4

In einem Reaktor wurden 202 g 70,5 gew.-%ige Schwefelsäure vorgelegt und auf 140°C erhitzt. Im Verlauf von 6 Stunden wurden 800 g 97,8 gew.-%iges 2,4-Dichlorbenzalchlorid und 37 g Wasser zugepumpt. Danach wurde das Reaktionsgemisch noch 2 Stunden bei 140°C nachgerührt. Der Umsatz war nach dieser Zeit quantitativ. Nach Zugabe von 25 ml Wasser wurden die resultierenden Phasen bei 120°C getrennt. Es wurden 198 g 69,4 gew.-%ige Schwefelsäure und nach Destillation der organischen Phase 579 g 97,6 gew.-%iger 2,4-Dichlorbenzaldehyd erhalten.

### Beispiel 5

In einem Reaktor wurden 1.000 g 60 gew.-%ige Schwefelsäure vorgelegt und auf 140°C erhitzt. Im Verlauf von 9,5 Stunden wurden gleichmäßig 3.200 g 98 Gew.-% reines 2,3-Dichlorbenzalchlorid zugepumpt. Dabei wurde die Temperatur so kontrolliert, daß sie 143°C nicht überstieg. Nach 3-stündigem Nachrühren bei max. 146°C war der Umsatz quantitativ. Dann wurden 252 g Wasser zudosiert und die resultierenden Phasen bei 110°C getrennt. Die Destillation der organischen Phase ergab 2,3-Dichlorbenzaldehyd in einer Ausbeute von 98 % der Theorie. Die Schwefelsäurephase enthielt fast quantitativ die eingesetzte Schwefelsäure.

### Beispiel 6

Es wurde gearbeitet wie in Beispiel 5, jedoch wurde statt 2,3-Dichlorbenzalchlorid eine entsprechende Menge 2,6-Dichlorbenzalchlorid eingesetzt. Die Dosierzeit betrugt 11,5 Stunden, die Reaktionstemperatur 140 bis 145°C und die Nachrührzeit 2,5 Stunden. Vor der Phasentrennung wurden 110 g Wasser zugesetzt und so bei 120°C 70 gew.-%ige Schwefelsäure abgetrennt (100 Gew.-% vom Einsatz). Ein aliquoter Teil der organischen Phase wurde einer Kurzweg-Destillation bei 30 mbar unterworfen, bei der die Sumpftemperatur max. 180°C und die Kopftemperatur max. 145°C betrug. Das Destillat entsprach 2.386 g 99 Gew.-% reinem 2,6-Dichlorbenzaldehyd und wies einen Schmelzpunkt von 71°C aufs Die abgetrennte Schwefelsäure wurde auf eine Konzentration von 60,5 Gew.-% verdünnt und bei einer Wiederholung des Beispiels wieder eingesetzt.

## Patentansprüche

1. Verfahren zur Herstellung von gegebenenfalls substituierten Benzaldehyden durch Verseifung der entsprechenden Benzalchloride mit Schwefelsäure, dadurch gekennzeichnet, daß man in Gegenwart von 100 bis 250 Mol-% Wasser, bezogen auf verseifbare Chloratome arbeitet und 1 bis 40 Gew.-% Schwefelsäure (gerechnet als 100%ige Schwefelsäure) bezogen auf Benzalchlorid einsetzt, Wasser in Gegenwart von Schwefelsäure bei 20 bis 160°C vorlegt, das Benzalchlorid und gegebenenfalls einen Teil des Wassers bei dieser Temperatur zumischt, nach Beendigung der Reaktion die Säurephase von der organischen Phase abtrennt, die Säurephase ganz oder teilweise in den nächsten Ansatz zurückführt und aus der organischen Phase den erhaltenen Benzaldehyd gewinnt.

2. Verfahren nach Anspruch 1, dadurch gekennzeichnet, daß man Benzalchloride der Formel (I) einsetzt 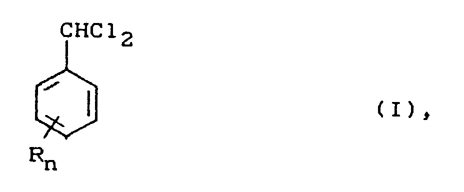 in der
R für Fluor, Chlor, Brom, NO₂ und/oder SO₃H und
n für Null, 1, 2, 3 oder 4 steht,
und Benzaldehyde der Formel (II) herstellt 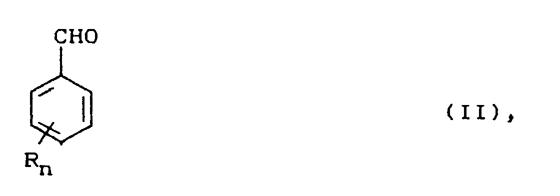 in der
R und n die bei Formel (I) angegebene Bedeutung haben.

3. Verfahren nach Ansprüchen 1 bis 2, dadurch gekennzeichnet, daß man die Schwefelsäure bei einer Temperatur von 40 bis 140°C vorlegt.

4. Verfahren nach Ansprüchen 1 bis 3, dadurch gekennzeichnet, daß die Temperatur während der Reaktion um nicht mehr als 35°C gegenüber der Temperatur vor der Zugabe des Benzalchlorids ansteigt und 180°C nicht übersteigt.

5. Verfahren nach Ansprüchen 1 bis 4, dadurch gekennzeichnet, daß man das Reaktionsgemisch nach Beendigung der Benzalchloridzugabe noch 1 bis 5 Stunden bei Reaktionstemperatur hält,

6. Verfahren nach Ansprüchen 1 bis 5, dadurch gekennzeichnet, daß man die Phasentrennung bei 20 bis 160°C vornimmt,

7. Verfahren nach Ansprüchen 1 bis 6, dadurch gekennzeichnet, daß man die abgetrennte Säurephase vollständig zum nächsten Ansatz zurückführt,

8. Verfahren nach Ansprüchen 1 bis 7, dadurch gekennzeichnet, daß man aus der abgetrennten organischen Phase den hergestellten Benzaldehyd durch Destillieren gewinnt.

9. Verfahren nach Ansprüchen 1 bis 8, dadurch gekennzeichnet, daß man 10 bis 90 gew.-%ige Schwefelsaure vorlegt.

## Claims

1. Process for the preparation of unsubstituted or substituted benzaldehydes by hydrolysis of the corresponding benzal chlorides using sulphuric acid, characterised in that the process is carried out in the presence of 100 to 250 mol % of water, relative to hydrolysable chlorine atoms, and 1 to 40% by weight of sulphuric acid (calculated as 100% pure sulphuric acid), relative to benzal chloride, is used, water in the presence of sulphuric acid is introduced at 20 to 160°C, the benzal chloride and, possibly, a part of the water are admixed at this temperature, after completion of the reaction the acid phase is separated off from the organic phase, the acid phase is wholly or partly returned to the next batch and the benzaldehyde obtained is recovered from the organic phase.

2. Process according to Claim 1, characterised in that benzal chlorides of the formula (I) are used 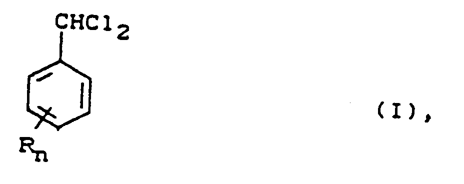 in which
R represents fluorine, chlorine, bromine, NO₂ and/or SO₃H and
n represents zero, 1, 2, 3 or 4,
and benzaldehydes of the formula (II) are prepared 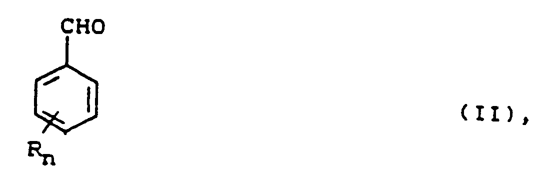 in which
R and n have the meaning given at formula (I).

3. Process according to Claims 1 and 2, characterised in that the sulphuric acid is introduced at a temperature of 40 to 140°C.

4. Process according to Claims 1 to 3, characterised in that the temperature during the reaction increases by not more than 35°C with respect to the temperature prior to the addition of the benzal chloride and does not exceed 180°C.

5. Process according to Claims 1 to 4, characterised in that the reaction mixture, after completion of the addition of benzal chloride, is kept for a further 1 to 5 hours at reaction temperature.

6. Process according to Claims 1 to 5, characterised in that the phase separation is undertaken at 20 to 160°C.

7. Process according to Claims 1 to 6, characterised in that the separated acid phase is completely returned to the next batch.

8. Process according to Claims 1 to 7, characterised in that the benzaldehyde prepared is recovered from the separated organic phase by distillation.

9. Process according to Claims 1 to 8, characterised in that 10 to 90% strength by weight of sulphuric acid is introduced.

## Revendications

1. Procédé de préparation de benzaldéhydes éventuellement substitués par saponification des chlorures de benzylidène correspondants par l'acide sulfurique, caractérisé en ce que l'on opère en présence de 100 à 250% en moles d'eau rapportés aux atomes de chlore saponifiables et de 1 à 40% en poids d'acide sulfurique (calculés comme acide sulfurique à 100%) rapportés au chlorure de benzylidène, que l'on introduit l'eau en présence d'acide sulfurique entre 20 et 160°C, que l'on mélange le chlorure de benzylidène et éventuellement une partie de l'eau à cette température, que, après l'achèvement de la réaction, l'on sépare la phase acide de la phase organique, que l'on recycle totalement ou partiellement la phase acide pour la réaction suivante et que l'on récupère le benzaldéhyde obtenu dans la phase organique.

2. Procédé selon la revendication 1, caractérisé en ce que l'on utilise des chlorures de benzylidène de formule (I) 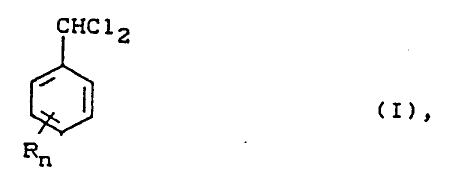 dans laquelle
R représente un atome de fluor, de chlore ou de brome, NO₂ et/ou SO₃H et
n représente 0, 1, 2, 3 ou 4,
pour préparer des benzaldéhydes de formule (II) 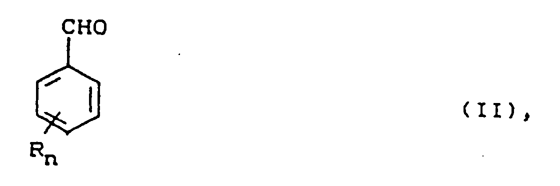 dans laquelle
R et n ont les significations indiquées pour la formule (I).

3. Procédé selon les revendications 1 et 2, caractérisé en ce que l'on introduit l'acide sulfurique à une température entre 40 et 140°C;

4. Procédé selon les revendications 1 à 3, caractérisé en ce que, pendant la réaction, la température n'augmente pas de plus de 35°C par rapport à la température avant l'addition du chlorure de benzylidène et qu'elle ne dépasse pas 180°C.

5. Procédé selon les revendications 1 à 4, caractérisé en ce que le mélange réactionnel est maintenu encore à la température de la réaction pendant 1 à 5 heures après l'achèvement de l'addition de chlorure de benzylidène.

6. Procédé selon les revendications 1 à 5, caractérisé en ce que l'on effectue la séparation des phases entre 20 et 160°C.

7. Procédé selon les revendications 1 à 6, caractérisé en ce que l'on recycle totalement la phase acide séparée pour la réaction suivante.

8. Procédé selon les revendications 1 à 7, caractérisé en ce que l'on récupère par distillation le benzaldéhyde préparé dans la phase organique séparée.

9. Procédé selon les revendications 1 à 8, caractérisé en ce que l'on utilise de l'acide sulfurique à une concentration entre 10 et 90% en poids.
